(19) Europäisches Patentamt
European Patent Office
Office européen des brevets

(11) Veröffentlichungsnummer: **0 036 836**
**B1**

(12) # EUROPÄISCHE PATENTSCHRIFT

(45) Veröffentlichungstag der Patentschrift: **12.09.84**

(51) Int. Cl.³: **C 09 B 57/04,** C 07 D 403/12, C 07 D 209/44, C 09 B 26/02

(21) Anmeldenummer: **81810084.4**

(22) Anmeldetag: **09.03.81**

(54) **Metallkomplexe von Isoindolinazinen, Verfahren zu deren Herstellung und Verwendung.**

(30) Priorität: **13.03.80 CH 1978/80**

(43) Veröffentlichungstag der Anmeldung: **30.09.81 Patentblatt 81/39**

(45) Bekanntmachung des Hinweises auf die Patenterteilung: **12.09.84 Patentblatt 84/37**

(84) Benannte Vertragsstaaten: **CH DE FR GB IT LI**

(56) Entgegenhaltungen:
**EP - A - 0 020 299**
**DE - A - 2 504 321**
**FR - A - 2 281 404**

***Die Akte enthält technische Angaben, die nach dem Eingang der Anmeldung eingereicht wurden und die nicht in dieser Patentschrift enthalten sind.***

(73) Patentinhaber: **CIBA-GEIGY AG, Postfach, CH-4002 Basel (CH)**

(72) Erfinder: **Iqbal, Abul, Dr., Im Schaiengarten 1, CH-4107 Ettingen (CH)**

**EP 0 036 836 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft 1:1-Metallkomplexe von Isoindolinazinen der Formel

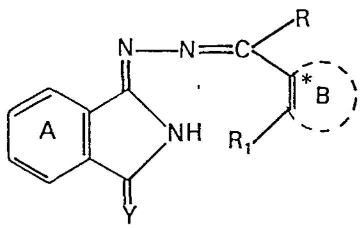

1

worin der Ring A noch weiter substituiert sein kann, R ein H-Atom, eine Alkyl- oder Arylgruppe, B einen isocyclischen oder heterocyclischen aromatischen oder einen alicyclischen Rest, $R_1$ die OH- oder SH-Gruppe und Y einen Rest der Formel

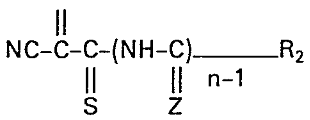

2

bedeutet, worin Z für ein O- oder S-Atom, n für die Zahl 1 oder 2 und $R_2$ für einen Alkyl-, Aralkyl-, Cycloalkyl- oder Arylrest oder eine gegebenenfalls durch Alkyl-, Cycloalkyl, Aralkyl- oder Arylrest substituierte Aminogruppe steht.

Die Isoindolinazine der Formel 1) können als Substituenten im Benzolrest A Halogenatome, beispielsweise 2-4 Chloratome, 1-2 Alkyl oder Alkoxygruppen mit 1 — 4 C, eine Phenyl-, Phenoxy-, Nitro- oder Benzoylaminogruppe oder eine Alkanoylaminogruppe mit 2-6 C enthalten, sind jedoch vorzugsweise unsubstituiert.

R steht beispielsweise für einen Phenyl- oder Naphthylrest, vorzugsweise für ein H-Atom oder eine Alkylgruppe mit 1-4 C, insbesondere die Methylgruppe.

B bedeutet beispielsweise einen Phenylen- oder Naphthylenrest, insbesondere aber einen gegebenenfalls anellierten 5 bis 6gliedrigen Heteroring enthaltend ein zum C*-Atom β-ständiges N-, O- oder S-Atom und gegebenenfalls ein weiteres N-Atom im Ring. B steht beispielsweise für einen Pyrazol-, Pyrimidin-, Chinolin- oder Cumarinring. $R_1$ bedeutet vorzugsweise die Hydroxygruppe. Bedeutet B einen alicyclischen Rest, dann vorzugsweise einen Cyclohexylrest.

Steht $R_2$ für einen Alkylrest, dann vorzugsweise für einen solchen mit 1-4 C. Bedeutet $R_2$ einen Cycloalkylrest, dann insbesondere den Cyclohexylrest. Bedeutet $R_2$ einen Arylrest, dann beispielsweise einen Naphthyl- und insbesondere Phenylrest.

Bedeutet $R_2$ eine Aminogruppe, so ist diese vorzugsweise durch eine Alkylgruppe mit 1-4 C, eine Cyclohexylgruppe, eine Phenyl- oder Benzylgruppe substituiert.

Bevorzugt sind Metallkomplexe der Formel

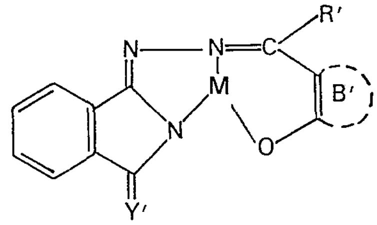

3

worin M Nickel oder Kupfer, R' H oder Methyl, B' einen Pyrazol-, Pyrimidin-, Chinolin- oder Cumarinrest und Y' einen Rest der Formel

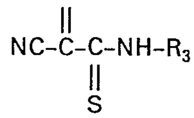

4

bedeutet, worin $R_3$ ein H-Atom, eine Alkylgruppe mit 1-4 C oder einen Rest der Formel

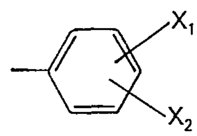

5

bedeutet, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylengruppen substituierte Phenoxy-, Phenylcarbamoyl- oder Phenylsulfamoylgruppe und $X_2$ ein H-, Chlor- oder Bromatom, eine Alkyl- oder Alkoxygruppe mit 1-4 C bedeuten.

Ebenfalls bevorzugt sind Metallkomplexe der Formel 3), worin Y' einen Rest der Formel

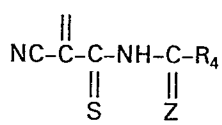

bedeutet, worin Z O oder S und $R_4$ eine Alkylgruppe mit 1-4 C, eine Cyclohexyl- oder Benzylgruppe oder eine Gruppe der Formel 5) bedeutet.

Die Formeln 1) und 3) stellen nur eine der verschiedenen isomeren Formen dar.

Die Metallkomplexe der Formel 1) erhält man, wenn man

a) ein Azin der Formel 1) mit metallabgebenden Mitteln behandelt oder

b) ein Hydrazon der Formel

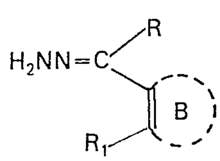

6

worin R, $R_1$ und B die angegebene Bedeutung haben, mit einem Isoindolinon der Formel

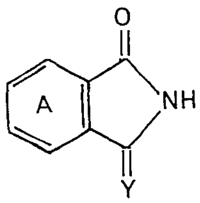

7

oder einem Amino-isoindolenin der Formel

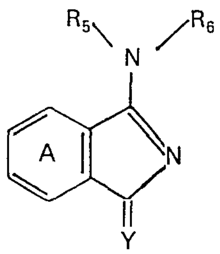

8

worin $R_5$ und $R_6$ H-Atome, Alkyl- Aryl- oder Heteroaryl-Gruppen oder $R_5$ und $R_6$ zusammen mit dem N-Atom einen heterocyclischen 5- oder 6-Ring bedeuten, A und Y die angegebene Bedeutung haben, in Gegenwart metallabgebender Mittel in einem polaren organischen Lösungsmittel erhitzt.

Sofern $R_5$ und $R_6$ Alkylreste bedeuten, dann vorzugsweise solche mit 1–6 C. Steht $R_6$ für einen Arylrest, dann vorzugsweise für einen gegebenenfalls durch Chloratome, Alkyl- oder Alkoxygruppen mit 1–4 C substituierten Phenylrest.

Zu den Azinen der Formel 1) gelangt man beispielsweise nach dem in der GB-PS 1.467.595 beschriebenen Verfahren durch Kondensation eines Hydrazons der Formel 6) mit einem Amino-isoindolenin der Formel 8) worin A und Y die angegebene Bedeutung haben, und $R_5 = R_6 = H$ ist.

Die Verbindung der Formel 8) erhält man durch Kondensation des Methin-imino-isoindolenins der Formel

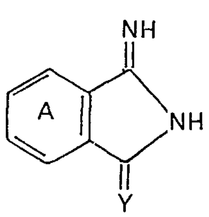

9

mit einem Amin der Formel

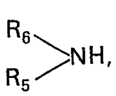

worin A, Y, $R_5$ und $R_6$ die angegebene Bedeutung haben. Die Verbindung der Formel 9) ihrerseits erhält man durch Umsetzung des Amino-imino-isoindolins der Formel

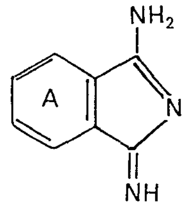

10

mit einer Verbindung der Formel

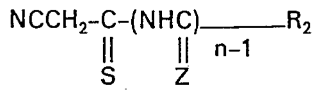

11

worin A, Z, n und $R_2$ die angegebene Bedeutung haben. Als Beispiele von Amino-imino-isoindolinen seien die auf S. 5 der GB-PS 1 465 595 aufgeführten erwähnt.

Als Beispiele von Verbindungen der Formel 11) seien erwähnt:

$NCCH_2CSCH_3$
$NCCH_2CSC_2H_5$
$NCCH_2CSCH_2CH_2CH_3$
$NCCH_2CSCH(CH_3)_3$
$NCCH_2CS(CH_2)_3CH_3$
$NCCH_2CSCH_2CH(CH_3)_2$
$NCCH_2CSCH_2C_6H_5$
$NCCH_2CS$-cyclohexyl
$NCCH_2$–CS–phenyl
$NCCH_2CS$-(o-, m- oder p)-chlorphenyl.
$NCCH_2CSNH_2$
$NCCH_2CSNHCH_3$
$NCCH_2CSNHC_2H_5$
$NCCH_2CSNHCH(CH_3)_2$
$NCCH_2CSNH(CH_2)_3CH_3$
$NCCH_2CSNHCH_2(CH_3)_2$
$NCCH_2CSNH$-cyclohexyl
$NCCH_2CSNHCH_2C_6H_5$
$NCCH_2CSNH$-phenyl
$NCCH_2CSNH$-(o-, m- oder p-)-chlorphenyl
$NCCH_2CSNH$-(o-, m- oder p-)-methylphenyl
$NCCH_2CSNH$-(o-, m- oder p-)-methoxyphenyl
$NCCH_2CSNH$-α-naphthyl.
$NCCH_2CSNHCOCH_3$
$NCCH_2CSNHCO$-phenyl.
$NCCH_2CSNHCONHC_2H_5$
$NCCH_2CSNHCONHC_6H_5$
$NCCH_2CSNHCSNHC_6H_5$

Die N-substituierten Thiocarbamoylacetonitril-Derivate lassen sich u.a. nach bekanntem Verfahren (A.D. Grabenko et al., ZH.Obshch. Khim. 32 (1962) 2 248 cf. Chem. Abstr. 58, 5546g, 7863e) durch Umsetzung von Cyanessigsäureestern mit dem entsprechenden Isothiocyanat und anschliessende Hydrolyse und Decarboxylierung herstellen.

Die Hydrazone der Formel 6) erhält man nach bekanntem Verfahren durch Umsetzen der Oxoverbindung der Formel

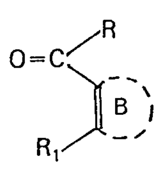

12

worin R, $R_1$ und B die angegebene Bedeutung haben, oder deren Iminen, vorzugsweise Anilen, mit Hydrazinhydrat.

Als Beispiele von Oxoverbindungen der Formel 11) seien die auf S. 11 und 12 der GB-PS 1 467 595 aufgeführten Aldehyde und Ketone erwähnt, ferner das 2-Formyl-5,5-dimethylcyclohexandion-1,3- und das 1-Phenyl-3-methyl-4-formyl-5-mercapto-pyrazol.

Die für den Weg b) als Ausgangsstoffe dienenden Isoindolinone der Formel 7) erhält man durch Umsetzen der Imino-isoindolinone der Formel

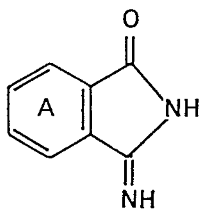

worin A die angegebene Bedeutung hat, eines Phthalodinitriles oder o-Cyanbenzoesäureesters, mit Verbindungen der Formel 11) nach bekannten Verfahren.

Als metallabgebende Mittel verwendet man vorzugsweise die Salze des Zinks, Cadmiums, Mangans, Kobalts, Eisens, insbesondere aber des Kupfers und des Nickels bzw. Mischungen solcher Metalle. Man verwendet zweckmässig die Formiate, Acetate oder Stearate dieser Metalle.

Die Umsetzungen erfolgen in einem polaren Lösungsmittel, insbesondere einem solchen hydrophiler Natur, beispielsweise einem Amid wie Dimethylformamid, Formamid, Dimethylacetamid oder N-Methylpyrrolidon, ferner Dimethylsulfoxid, Acetonitril oder einem Alkohol wie beispielsweise Äthylcellosolve. Es kann auch ein Gemisch polarer Lösungsmittel verwendet werden.

Die Umsetzungstemperatur liegt zweckmässig zwischen 100–200°C.

Die Isolierung des erhaltenen Metall-Komplexes erfolgt wie üblich durch Filtration. Das Nutschgut wird mit Lösungsmittel gut gewaschen. Es wird in ausgezeichneter Ausbeute und Reinheit erhalten und kann ohne weitere Reinigung in feinverteilter Form zum Färben von hochmolekularem organischen Material verwendet werden, z.B. Celluloseäthern und -estern, wie Äthylcellulose, Acetylcellulose, Nitrocellulose, Polyamiden bzw. Polyurethanen oder Polyestern, natürlichen Harzen oder Kunstharzen, z.B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyäthylen, Polypropylen, Polyacrylnitril, Polyacrylsäureester, thermoplastische oder härtbare Acrylharze, Gummi, Casein, Silikon und Silikonharzen, einzeln oder in Mischungen. Die erwähnten hochmolekularen Verbindungen können als plastische Massen, Schmelzen oder in Form von Spinnlösungen, Lacken oder Druckfarben vorliegen. Je nach Verwendungszweck erweist es sich als vorteilhaft, die neuen Pigmente als Toner oder in Form von Präparaten zu verwenden.

Das Pigment kann in der Form, wie es in der Synthese anfällt, oder in leicht gemahlener Form eingesetzt werden und liefert dann opake Ausfärbungen. Man kann es aber auch einer intensiveren Mahlung unterziehen und erhält dann transparente Ausfärbungen, wie beispielsweise farbstarke Metalleffektlackierungen.

Anreibungen der Pigmente in Lacken zeichnen sich durch ein günstiges Fliessverhalten aus.

Die erhaltenen Färbungen beispielsweise in Kunststoffen, Fasern und Lacken zeichnen sich durch grosse Farbstärke, hohe Farbtonreinheit, gute Dispergierbarkeit, gute Überlackier-, Migrations-, Hitze-, Licht- und Wetterechtheit sowie durch einen guten Glanz aus.

Von nächstvergleichbaren Metallkomplexen der DE-A 2 504 321 unterscheiden sich die erfindungsgemässen dadurch, dass Y anstelle eines Cyancarbamoylmethylen- einen Cyanthiocarbamoylmethylenrest bedeutet. Gegenüber diesen bekannten Pigmenten zeichnen sich die erfindungsgemässen durch eine bessere Hitzebeständigkeit aus.

In den nachfolgenden Beispielen bedeuten die Prozente Gewichtsprozente und die Grade Celsiusgrade.

Beispiel 1

12,21 g (0,04 Mol) 1-(Cyano-phenylthiocarbamoylmethylen)-3-oxo-isoindolin, hergestellt aus 1-Imino-3-oxo-isoindolin und Cyanthioacetanilid, 10,03 g (0,04 Mol) 1-p-Chlorphenyl-3-methyl-4-hydrazinomethylen-5-pyrazolon und 10,5 g (0,042 Mol) Nickelacetat·$4H_2O$ werden nacheinander in 400 ml Dimethylformamid angeschlämmt. Die resultierende Suspension wird auf 120°C erhitzt und bei derselben Temperatur 2 h gerührt. Anschliessend wird das Gemisch auf 80°C abgekühlt und der dick ausgefallene Metallkomplex abfiltriert. Man wäscht mit Dimethylformamid und Äthanol nach und trocknet das Nutschgut unter Vakuum bei 80°C. Auf diese Weise erhält man 22,2 g (93,3% der Theorie) des 1:1-Nickelkomplexes der Formel

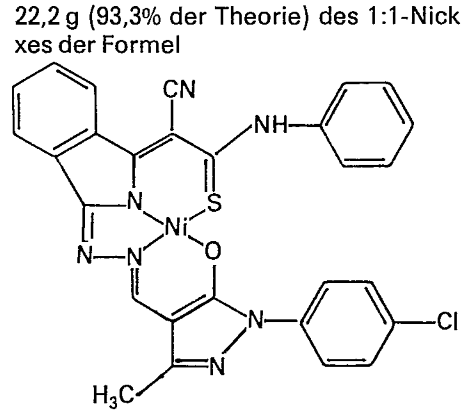

Mikroanalyse: $C_{29}H_{18}ClN_7OSNi$ MG 594,72

ber.* C 54,8% H 3,3% N 16,0% S 5,23% Ni 9,98%
gef. C 54,7% H 3,1% N 16,5% S 5,5 % Ni 9,43%

* unter Berücksichtigung der gefundenen $H_2O$-Menge von 2,9%

Das obige Metallkomplex-Pigment färbt Kunststoffe und Lacke in roten Tönen von ausgezeichneten Echtheiten.

Beispiel 2

1,95 g (0,008 Mol) 2-Anilinomethylen-5,5-dimethylcyclohexan-1,3-dion werden in 50 ml Dimethylformamid gelöst, mit 0,4 ml Hydrazinhydrat versetzt und anschliessend 1 Stunde bei Raumtemperatur gerührt. Man gibt 2,1 g (0,0084 Mol) Nickelacetat$\cdot 4H_2O$ hinzu und erwärmt auf 60°C. Die resultierende Suspension wird mit 2,44 g (0,008 Mol) 1-(Cyan-N-phenylthiocarbamoylmethylen)-3-isoindolinon versetzt, auf 120°C erwärmt und bei derselben Temperatur 1 Stunde gerührt. Der ausgefallene Metallkomplex wird nach Abkühlung des Reaktionsgemisches auf 80°C abfiltriert, mit Dimethylformamid und Äthanol gewaschen und schliesslich über Nacht bei 80°C unter Vakuum getrocknet. Man erhält 3,5 g (83% der Theorie) eines roten Pigments der Zusammensetzung

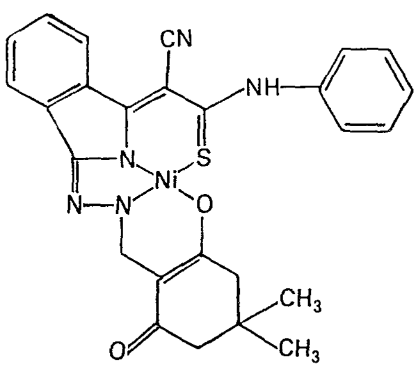

Mikroanalyse: $C_{26}H_{21}N_5O_2SNi$ MG 526

ber. C 59,34% H 4,02% N 13,31% S 6,09% Ni 11,16%
gef. C 59,2% H 4,2% N 13,5% S 6,5% Ni 11,3%

Der obige Metallkomplex färbt Kunststoffe und Lacke in orangen Tönen von ausgezeichneten Echtheiten.

Beispiel 3

Verwendet man im Beispiel 2 anstelle des 1-(Cyan-N-phenyl-thiocarbamoylmethylen)-3-isoindolinons 1-(Cyan-thiocarbamoylmethylen)-3-isoindolinon, so erhält man bei analoger Arbeitsweise 2,3 g (64% der Theorie) eines orangeroten Komplexes der Zusammensetzung

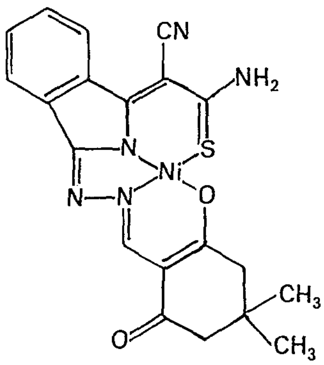

Mikroanalyse: $C_{20}H_{17}N_5O_2SNi$ MG 450

ber. C 53,36% H 3,81% N 15,56% S 7,12% Ni 13,04%
gef. C 53,5% H 3,8% N 15,8% S 7,1% Ni 13,0%

Das obige Pigment färbt Kunststoffe und Lacke in orangen Tönen von hohem Echtheitsniveau.

Beispiel 4

1,72 g (0,007 Mol) 1-Phenyl-3-carbamoyl-4-hydrazinomethylen-pyrazolon-5 werden mit 1,83 g (0,00735 Mol) Nickelacetat$\cdot 4H_2O$ in 30 ml N-Methylpyrrolidon suspendiert und anschliessend auf 60°C erwärmt. Man gibt 2,14 g (0,007 Mol) 1-(Cyan-N-phenylthiocarbamoylmethylen)-3-isoindolinon hinzu, erhitzt auf 115°C und lässt das Gemisch bei derselben Temperatur 2 Stunden lang ausreagieren. Anschliessend wird auf 80°C abgekühlt und das ausgefallene Produkt abfiltriert. Nach Waschen mit N-Methylpyrrolidon und Äthanol sowie Trocknung bei 80°C unter Vakuum erhält man 2,6 g (67% der Theorie) eines weinroten Metallkomplexes der Struktur

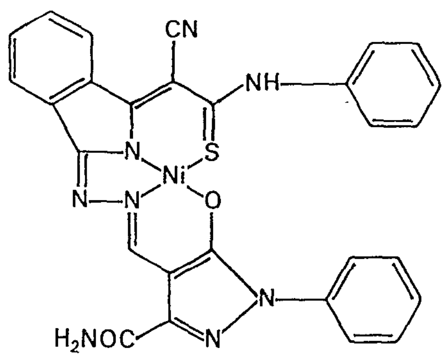

Mikroanalyse: $C_{28}H_{18}N_8O_2SNi$ MG 589

ber. C 57,07% H 3,08% N 19,01% S 5,44% Ni 9,96%
gef. C 57,2% H 3,2% N 19,2% S 5,3% Ni 9,8%

In Kunststoffen und Lacken verarbeitet liefert das obige Pigment rote Töne von ausgezeichneten Echtheiten.

Beispiel 5

1,3 g (0,006 Mol) 2,4-Dihydroxy-3-acetylchinolin-hydrazon und 1,57 g (0,0063 Mol) Nickelacetat$\cdot 4H_2O$ werden in 50 ml Dimethylformamid angeschlämmt. Nach Erwärmen auf 60°C gibt man unter Rühren 1,82 g (0,006 Mol) 1-(Cyan-thiocarbamoylmethylen)-5,6-dichlor-3-isoindolinon hinzu, erhitzt weiter auf 105°C und lässt 2 Stunden bei derselben Temperatur ausreagieren. Anschliessend wird auf 80°C abgekühlt und der ausgefallene Metallkomplex abfiltriert, mit Dimethylformamid und Äthanol gewaschen und über Nacht bei 80°C unter Vakuum getrocknet. Man erhält 3,2 g (96% der Theorie) eines roten Metallkomplexes mit der Zusammensetzung $C_{22}H_{12}Cl_2N_6O_2SNi$ und der Struktur

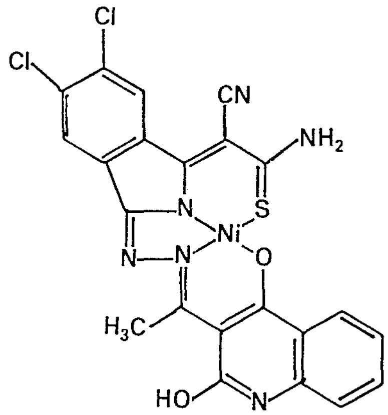

Mikroanalyse: $C_{22}H_{12}Cl_2N_6O_2SNi$ MG 554

ber. C 47,69% H 2,18% N 15,17% S 5,79% Cl 12,80% Ni 10,60%
gef. C 47,2% H 2,4% N 15,3% S 5,5% Cl 12,4% Ni 10,3%

Das obige Pigment färbt Kunststoffe und Lacke in blaustichig roten Tönen von ausgezeichneten Echtheiten.

Beispiel 6

In eine Lösung von 2,35 g (0,008 Mol) 1-Phenyl-3-methyl-4-anilinomethylen-5-thiopyrazolon in 50 ml Dimethylformamid gibt man 0,4 ml Hydrazinhydrat (0,008 Mol) hinzu und rührt das Gemisch bei Raumtemperatur 1 Stunde lang. Nach weiterer Zugabe von 2,1 g (0,0084 Mol) Nickelacetat·$4H_2O$ erwärmt man auf 60°C und versetzt das Reaktionsgemisch anschliessend mit 2,44 g (0,008 Mol) 1-(Cyan-N-phenylthiocarbamoylmethylen)-3-isoindolinon. Man lässt das Gemisch bei 120°C 1 Stunde ausreagieren, kühlt anschliessend auf 80°C ab und filtriert. Der Filterrückstand wird mit Dimethylformamid und Äthanol gewaschen und über Nacht bei 80°C unter Vakuum getrocknet. Man erhält 2,2 g (48% der Theorie) eines rötlich braunen Metallkomplexes der Zusammensetzung

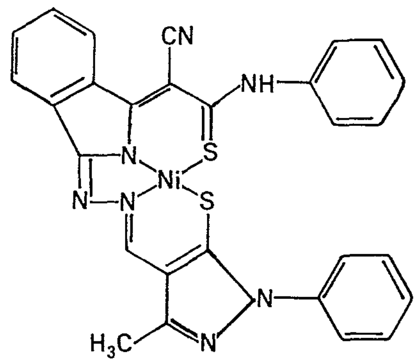

Mikroanalyse: $C_{28}H_{19}N_7S_2Ni$ MG 576

ber. C 58,35% H 3,32% N 17,01% S 11,13% Ni 10,19%
gef. C 57,8% H 3,4% N 17,1% S 11,1% Ni 10,3%

Beispiele 7–35

Analog den Beispielen 1–6 erhält man weitere 1:1-Nickelkomplexe, wenn man das Hydrazon der in Kolonne 2 der Tabelle angegebenen Oxoverbindungen mit dem Isoindolinon der Formel

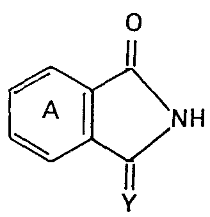

kondensiert, welch letzteres erhalten wurde durch Kondensation des in Kolonne 3 erwähnten 3-Imino-isoindolinon mit der in Kolonne 4 aufgeführten Verbindungen $YH_2$. Kolonne 5 gibt die Nuance in PVC.

Tabelle 1

| Beispiel Nr. | Oxoverbindung | Isoindolinon | $YH_2$ | Nuance in PVC |
|---|---|---|---|---|
| 7 | 3-Acetyl-2,4-dihydroxychinolin | 3-Iminoisoindolinon | Cyanmethylthio-carb-p-chlor-anilid | rot |
| 8 | 3-Acetyl-2,4-dihydroxychinolin | 3-Iminoisoindolinon | Cyanmethylthio-carb-p-toluidid | rot |
| 9 | 3-Acetyl-2,4-dihydroxychinolin | 3-Iminoisoindolinon | Cyanmethylthio-carb-p-anisidid | rot |
| 10 | 1-p-Chlorphenyl-3-methyl-4-formylpyrazolon-5 | 3-Iminoisoindolinon | Cyanmethyl-thiocarbanilid | rot |
| 11 | 3-Acetyl-2,4-dihydroxychinolin | 3-Iminoisoindolinon | Cyanmethyl-thiocarbanilid | rot |
| 12 | 3-Acetyl-2,4-dihydroxychinolin | 3-Iminoisoindolinon | Cyanmethyl-thiocarbanilid | rot |
| 13 | 5-Acetyl-2,4,6-trihydroxypyrimidin | 3-Iminoisoindolinon | Cyanmethyl-thiocarbanilid | orange |
| 14 | 3-Acetyl-4-hydroxy-cumarin | 3-Iminoisoindolinon | Cyanmethyl-thiocarbanilid | rot |
| 15 | 1-(2',4',6'-Trichlorphenyl)-3-methyl-4-formylpyrazolon-5 | 3-Iminoisoindolinon | Cyanmethyl-thiocarbanilid | rot |

Tabelle 1 (Fortsetzung)

| Beispiel Nr. | Oxoverbindung | Isoindolinon | $YH_2$ | Nuance in PVC |
|---|---|---|---|---|
| 16 | 1-(3',4'-Dichlorphenyl)-3-methyl-4-acetyl-pyrazolon-5 | 3-Iminoisoindolinon | Cyanmethyl-thiocarbanilid | rot |
| 17 | 3-Acetyl-2,4-dihydroxychinolin | 3-Iminoisoindolinon | Cyanmethyl-thiocarbanilid | rot |
| 18 | 5-Acetyl-2,4,6-trihydroxy-pyrimidin | 3-Iminoisoindolinon | Cyanmethyl-thiocarbanilid | orange |
| 19 | 3-Acetyl-4-hydroxycumarin | 3-Iminoisoindolinon | Cyanmethyl-thiocarbanilid | rot |
| 20 | 1-Phenyl-5-acetyl-4,6-dihydroxy-pyrimidin | 3-Iminoisoindolinon | Cyanmethyl-thiocarbanilid | rot |
| 21 | 3-Acetyl-2,4-dihydroxychinolin | 3-Iminoisoindolinon | Cyanmethyl-thiocarbbutylamid | rot |
| 22 | 3-Acetyl-4-hydroxy-cumarin | 3-Iminoisoindolinon | Cyanmethyl-thiocarbbutylamid | orange |
| 23 | 3-Acetyl-4-hydroxy-cumarin | 3-Iminoisoindolinon | Cyanmethyl-thiocarbbutylamid | scharlach |
| 24 | 1-p-Chlorphenyl-3-methyl-4-acetyl-pyrazolon-5 | 3-Iminoisoindolinon | Cyanmethyl-thiocarbbutylamid | orange |
| 25 | 1-Methyl-3-acetyl 4-hydroxy-chinolon-2 | 3-Iminoisoindolinon | Cyanmethyl-thiocarbbutylamid | rot |
| 26 | 3-Acetyl-2,4-dihydroxy-chinolin | 3-Imino-4,5,6,7-tetrachlor-isoindolinon | Cyanmethyl-thiocarbbutylamid | violett |
| 27 | 3-Acetyl-2,4-dihydroxy-chinolin | 3-Imino-5,6-dichlor-isoindolinon | Cyanmethyl-thiocarbbutylamid | bordeaux |
| 28 | 1-p-Tolyl-3-carbamoyl-4-formylpyrazolon-5 | 3-Iminoisoindolinon | Cyanmethyl-thiocarbamid | orange |
| 29 | 1-p-Tolyl-3-carbamoyl-4-formylpyrazolon-5 | 3-Iminoisoindolinon | Cyanmethyl-thiocarbanilid | rot |
| 30 | 1-Phenyl-3-acetyl-amino-4-formylpyrazolon-5 | 3-Iminoisoindolinon | Cyanmethyl-thiocarbanilid | braun |
| 31 | 1-p-Chlorphenyl-3-carbamoyl-4-formylpyrazolon-5 | 3-Iminoisoindolinon | Cyanmethyl-thiocarbamid | orange |
| 32 | 1-p-Chlorphenyl-3-methyl-4-formylpyrazolon-5 | 4,6-Dimethoxy-5,7-dichlor-3-iminoisoindolinon | Cyanmethyl-thiocarbamid | rot |
| 33 | 1-p-Chlorphenyl-3-methyl-4-formylpyrazolon-5 | 5,6-Dichlor-3-iminoisoindolinon | Cyanmethyl-thiocarbamid | rot |
| 34 | 3-Acetyl-2,4-dihydroxychinolin | 4,6-Dimethoxy-5,7-dichlor-3-iminoisoindolinon | Cyanmethyl-thiocarbamid | bordeaux |
| 35 | 3-Acetyl-4-hydroxycumarin | 4,6-Dimethoxy-5,7-dichlor-3-iminoisoindolinon | Cyanmethyl-thiocarbamid | rot |

Beispiel 36

In einem Laboratoriums-Kneter von 250 Vol.-Teilen Inhalt legt man 25 Teile des gemäss Beispiel 1 hergestellten Pigmentes, 100 Teile feingemahlenes Natriumchlorid und 30 Teile Diacetonalkohol vor. Man knetet die Mischung während 5 Stunden unter Kühlung und trägt sie dann in 4000 Vol.-Teile Wasser ein. Natriumchlorid sowie Diacetonalkohol gehen in Lösung, das Pigment fällt aus. Man filtriert die Suspension, wäscht das Nutschgut gründlich mit Wasser und trocknet es im Vakuumtrockenschrank bei 80°.

Beispiel 37

1,09 g (0,005 Mol) 3-Acetyl-2,4-dihydroxy-chinolin-hydrazon und 1,31 g (0,00525 Mol) Nickelacetat·$4H_2O$ werden in 100 ml Dimethylformamid angeschlämmt. Nach Erwärmen auf 60°C gibt man unter Rühren 1,78 g (0,005 Mol) 1-(Cyano-α-naphthyl-thiocarbamoyl-methylen)-3-oxo-isoindolin, hergestellt aus Cyanthioessigsäure-α-naphthylamid und 1-Imino-3-oxo-isoindolin, hinzu. Das Reaktionsgemisch wird unter Rühren auf 120°C erhitzt und bei derselben Temperatur 2 Stunden gerührt. Anschliessend wird auf 100°C abgekühlt und der ausgefallene Metallkomplex abfiltriert, mit Dimethylformamid und Äthanol gewaschen und über Nacht bei 80°C unter Vakuum getrocknet. Man erhält 2,9 g (97% der Theorie) eines roten Metallkomplexes der Formel

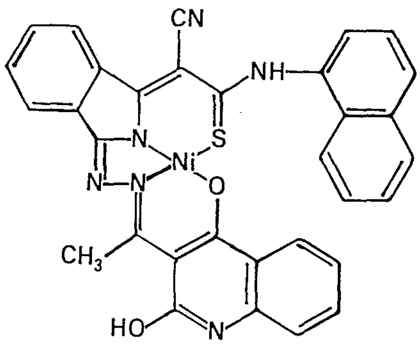

Mikroanalyse: $C_{31}H_{20}N_6O_2SNi$ MG 599 g

ber. C 62,1% H 3,4% N 14,0% S 5,4% Ni 9,8%
gef. C 62,7% H 3,7% N 13,9% S 5,7% Ni 9,6%

Der obige Metallkomplex färbt Kunststoffe und Lacke in roten Tönen von ausgezeichneten Echtheiten.

Beispiele 38–47

Analog dem Beispiel 41 erhält man durch Umsetzung eines Isoindolinons der Formel

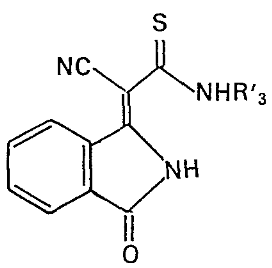

mit einem Hydrazon der Formel

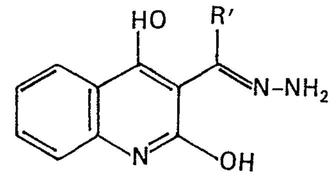

in Gegenwart von Nickelacetat·$4H_2O$ weitere in Tabelle 2 aufgeführte 1:1-Nickelkomplexe der Formel

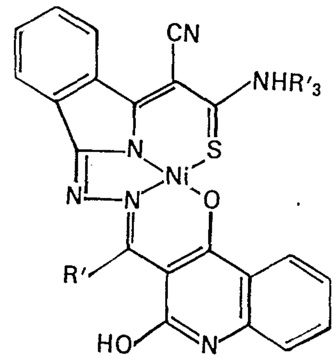

wobei R′ und $R'_3$ die in Kolonne 2 bzw. 3 angegebene Bedeutung haben. Kolonne 4 gibt die Nuance in PVC wieder.

| Beispiel Nr. | R′ | $R'_3$ | Nuance in PVC |
|---|---|---|---|
| 38 | 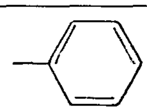 | H | rot |
| 39 | 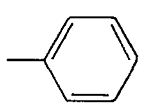 | 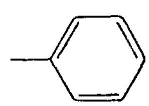 | blaurot |
| 40 | $CH_3$– | 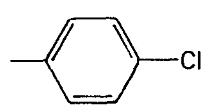 | blaurot |
| 41 | $CH_3$– | 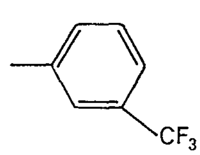 | blaurot |
| 42 | $CH_3$– | 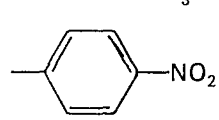 | blaurot |
| 43 | $CH_3$– | 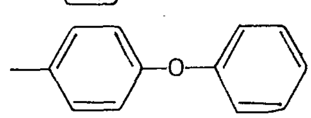 | blaurot |

| Beispiel Nr. | R′ | $R'_3$ | Nuance in PVC |
|---|---|---|---|
| 44 | $CH_3$- | 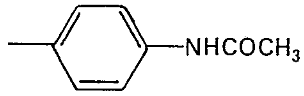 | blaurot |
| 45 | $CH_3$- | 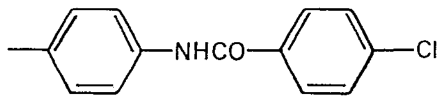 | blaurot |
| 46 | $CH_3$- | 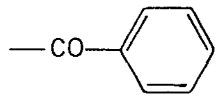 | blaurot |
| 47 | $CH_3$- | 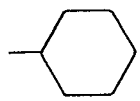 | rot |

Beispiel 48

21,74 g (0,1 Mol) 3-Acetyl-2,4-dihydroxychinolin-hydrazon und 26,11 g (0,105 Mol) Nickelacetat·$4H_2O$ werden in 400 ml Äthylcellosolve angeschlämmt und auf 70°C erwärmt. Hierauf gibt man 22,93 g (0,1 Mol) der Isoindolinon-Verbindung der Formel

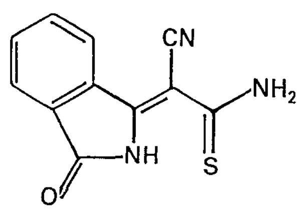

hergestellt aus Cyanmethylthiocarbamid und 1-Imino-3-oxo-isoindolin, hinzu und erhitzt auf 110°C. Das Gemisch wird bei derselben Temperatur 1½ Stunden lang gerührt, anschliessend auf 80°C abgekühlt und filtriert. Das Nutschgut wird mit Äthylcellosolve und Äthanol gewaschen und über Nacht bei 80°C unter Vakuum getrocknet. Man erhält 48,2 g (99,3% der Theorie) des 1:1-Nikkelkomplexes der Formel

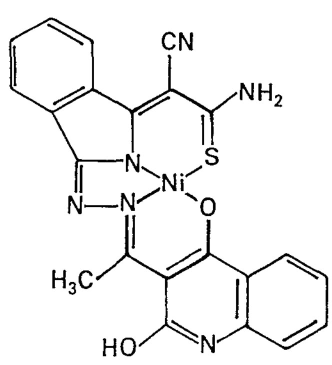

(nur eine der möglichen isomeren bzw. tautomeren Formen wurde berücksichtigt) als bordeauxrotes Pulver.

Mikroanalyse: $C_{22}H_{14}N_6O_2SNi$ Mol.-Gew. 485,2

ber.* C 54,26% H 2,94% N 17,26% S 6,58% Ni 12,06%

gef. C 54,0% H 3,0% N 17,2% S 6,6% Ni 11,9% Ni $H_2O$ 0,4%

* berechnet unter Berücksichtigung der gefundenen Wassermenge (0,4%)

Der obige Metallkomplex färbt Kunststoffe und Lacke in bordeaux-roten Tönen von ausgezeichneten Echtheiten.

Beispiel 49

65 Teile stabilisiertes Polyvinylchlorid, 35 Teile Dioctylphthalat und 0,2 Teil des gemäss Beispiel 36 erhaltenen Pigmentes werden miteinander verrührt und dann auf einem Zweiwalzenkalander während 7 Minuten bei 140° hin- und hergewalzt. Man erhält eine rot gefärbte Folie von sehr guter Licht- und Migrationsechtheit.

Beispiel 50

10 g Titandioxyd und 2 g des nach Beispiel 1 hergestellten Pigments werden mit 88 g einer Mischung von 26,4 g Kokosalkydharz, 24,0 g Melamin-Formaldehydharz (50% Festkörpergehalt), 8,8 g Äthylenglykolomonomethyläther und 28,8 g Xylol während 48 Stunden in einer Kugelmühle vermahlen.

Wird dieser Lack auf eine Aluminiumfolie gespritzt, 30 Minuten bei Raumtemperatur vorgetrocknet und dann während 30 Minuten bei 120°C eingebrannt, dann erhält man eine blaustichige Rotlackierung, die sich bei guter Farbstärke durch eine sehr gute Überlackier-, Licht- und Wetterechtheit auszeichnet.

Beispiel 51

4 Teile des fein verteilten Pigments gemäss Beispiel 36 werden in 20 Teilen Lösungsmittel der folgenden Zusammensetzung eingerührt: 50 Teile Solvesso 150 (Gemisch aromatischer Kohlenstoffe), 15 Teile Butylacetat, 5 Teile Exkin II (Verlaufmittel auf Ketoximbasis), 25 Teile Methyl-Isobutylketon, 5 Teile Silikonöl (1% in Solvesso 150).

Nachdem die vollständige Feinverteilung erreicht ist (je nach Art des Rührens in ca. 15-60 Minuten), werden die Bindemittel zugesetzt, nämlich 48,3 Teile Baycryl L 530 (Acrylharz) (51% in Xylol/Butanol 3:1) und 23,7 Teile Maprenal TTX (Melaminharz) (55% in Butanol).

Nach kurzem Homogenisieren wird der Lack nach üblichen Methoden wie Spritzen und Tauchen oder speziell zur kontinuierlichen Beschichtung von Metallblechen im «Coil-Coating»-Verfahren appliziert und eingebrannt (Einbrennen 30 Minuten, 130°). Die erhaltenen blaustichig roten Lackierungen zeichnen sich aus durch sehr guten Verlauf, hohen Glanz und ausgezeichnete Feinverteilung des Pigments, sowie durch ausgezeichnete Wetterechtheiten.

Beispiel 52

Verfährt man wie in Beispiel 36 beschrieben, fügt jedoch der Knetmasse 2,78 Teile Staybelite Resin (HERCULES) zu, so erhält man ein Pigment, enthaltend 10% Harz, das sich auszeichnet durch leichtere Einarbeitbarkeit und bessere Verteilbarkeit.

Die im Vorstehenden genannten Warenzeichen werden als solche anerkannt.

**Patentansprüche**

1. 1:1-Metallkomplexe von Azinen der Formel

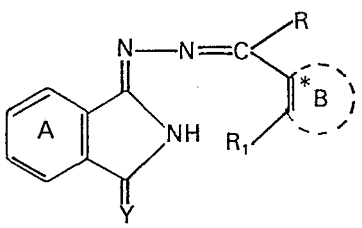

1

worin der Ring A noch weiter substituiert sein kann, R ein H-Atom, eine Alkyl- oder Arylgruppe, B einen isocyclischen oder heterocyclischen aromatischen oder einen alicyclischen Rest, $R_1$ die OH- oder SH-Gruppe und Y einen Rest der Formel

```
   ||
NC-C-C-(NH-C)———R2
     ||    || n-1
     S     Z
```

2

bedeutet, worin Z für ein O- oder S-Atom, n für die Zahl 1 oder 2 und $R_2$ für einen Alkyl-, Aralkyl-, Cycloalkyl- oder Arylrest oder eine gegebenenfalls durch Alkyl-, Cycloalkyl, Aralkyl- oder Arylrest substituierte Aminogruppe steht.

2. Metallkomplexe der Formel

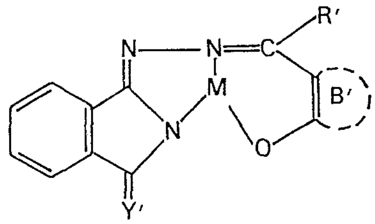

3

worin M Nickel oder Kupfer, R′ H oder Methyl, B′ einen Pyrazol-, Pyridin-, Pyrimidin-, Chinolin- oder Cumarinrest und Y′ einen Rest der Formel

```
   ||
NC-C-C-NH-R3
     ||
     S
```

4

bedeutet, worin $R_3$ ein H-Atom, eine Alkylgruppe mit 1-4 C oder einen Rest der Formel

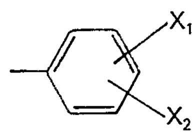

5

bedeutet, worin $X_1$ ein H-, Chlor- oder Bromatom, eine Nitro-, Trifluormethyl-, Carbamoyl- oder Sulfamoylgruppe, eine Alkyl-, Alkoxy- oder Alkylsulfamoylgruppe mit 1-4 C, eine Alkanoylamino-, Alkylcarbamoyl- oder Alkoxycarbonylgruppe mit 2-6 C, eine gegebenenfalls durch Chlor- oder Bromatome oder Methylengruppen substituierte Phenoxy-, Phenylcarbamoyl- oder Phenylsulfamoylgruppe und $X_2$ ein H-, Chlor- oder Bromatom, eine Alkyl- oder Alkoxygruppe mit 1-4 C bedeuten.

3. Metallkomplexe gemäss Anspruch 1, worin Y einen Rest der Formel

```
   ||
NC-C-C-NH-C-R4
     ||   ||
     S    Z
```

6

bedeutet, worin Z ein O- oder S-Atom und $R_4$ eine Alkylgruppe mit 1-4 C, eine Cyclohexylgruppe, eine Benzylgruppe oder eine Gruppe der Formel

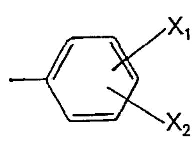

5

bedeutet, worin $X_1$ und $X_2$ die im Anspruch 2 angegebene Bedeutung haben.

4. Verfahren zur Herstellung von 1:1-Metallkomplexen gemäss Anspruch 1, dadurch gekennzeichnet, dass man

a) ein Azin der Formel 1) mit metallabgebenden Mitteln behandelt oder

b) ein Hydrazon der Formel

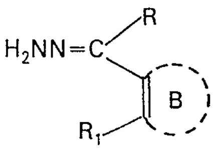

6

worin R, $R_1$ und B die in Anspruch 1 angegebene Bedeutung haben, mit einem Isoindolinon der Formel

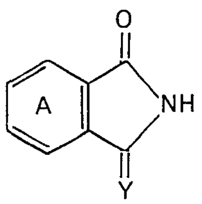

7

oder einem Amino-isoindolenin der Formel

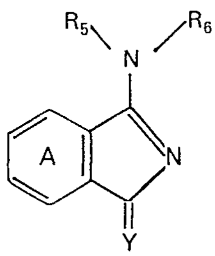

8

worin A und Y die in Anspruch 1 angegebene Bedeutung haben, und $R_5$ und $R_6$ H-Atome, Alkyl- oder Heteroarylreste bedeuten oder $R_5$ und $R_6$ zusammen mit dem N-Atom einen 5- bis 6-gliedrigen Heteroring bedeuten, in Gegenwart metallabgebender Mittel in einem polaren organischen Lösungsmittel erhitzt.

5. Verfahren gemäss Anspruch 4, dadurch gekennzeichnet, dass man von einem Isoindolinon der Formel 7) oder einem Isoindolenin der Formel 8) ausgeht, worin $R_5$ und $R_6$ die in Anspruch 4 angegebene Bedeutung haben und Y' einen Rest der Formel

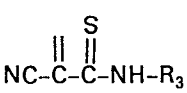

bzw.

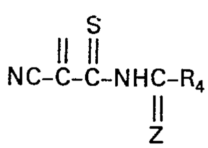

bedeutet, worin $R_3$, $R_4$ und Z die in Anspruch 2 bzw. 3 angegebene Bedeutung haben.

6. Verfahren zum Färben von hochmolekularem organischem Material, gekennzeichnet durch die Verwendung der Metallkomplexe gemäss Anspruch 1.

7. Hochmolekulares organisches Material enthaltend einen Metallkomplex gemäss Anspruch 1.

## Claims

1. 1:1 Metal complexes of azines of the formula

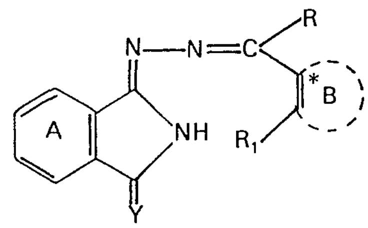

1

in which the ring A can be further substituted, R is an H atom or an alkyl or aryl group, B is an isocyclic or heterocyclic aromatic radical or an alicyclic radical, $R_1$ is the OH or SH group and Y is a radical of the formula

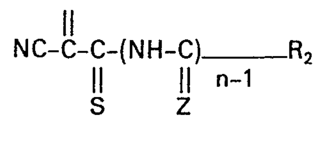

2

in which Z is an O or S atom, n is the number 1 or 2 and $R_2$ is an alkyl, aralkyl, cycloalkyl or aryl radical or an amino group which is unsubstituted or substituted by an alkyl, cycloalkyl, aralkyl or aryl radical.

2. Metal complexes according to claim 1 of the formula

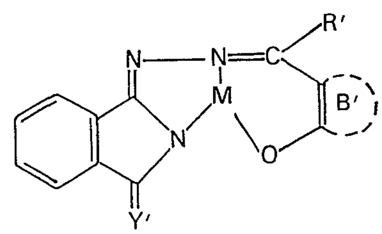

3

in which M is nickel or copper, R' is H or methyl, B' is a pyrazole, pyridine, pyrimidine, quinoline or coumarin radical and Y' is a radical of the formula

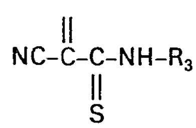

4

in which $R_3$ is an H atom, an alkyl group having 1-4 C or a radical of the formula

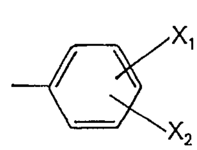

5

in which $X_1$ is an H, chlorine or bromine atom, a nitro, trifluoromethyl, carbamoyl or sulfamoyl group, an alkyl, alkoxy or alkylsulfamoyl group having 1-4 C, an alkanoylamino, alkylcarbamoyl or alkoxycarbonyl group having 2-6 C, or a phenoxy, phenylcarbamoyl or phenylsulfamoyl group which is unsubstituted or substituted by chlorine or bromine atoms or methylene groups, and $X_2$ is an H, chlorine or bromine atom or an alkyl or alkoxy group having 1-4 C.

3. Metal complexes according to claim 1, in which Y is a radical of the formula

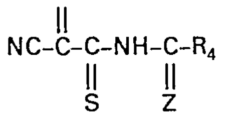

6

in which Z is an O or S atom and $R_4$ is an alkyl group having 1-4 C, a cyclohexyl group, a benzyl group or a group of the formula

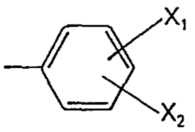

5

in which $X_1$ and $X_2$ are as defined in claim 2.

4. Process for the preparation of 1:1 metal complexes according to claim 1, which comprises a) treating an azine of the formula 1) with metal donors or b) heating a hydrazone of the formula

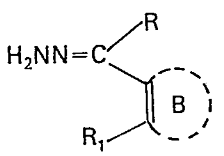

6

in which R, $R_1$ and B are as defined in claim 1, with an isoindolinone of the formula

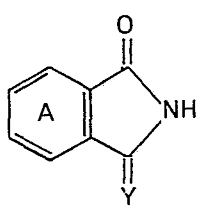

7

or an amino-isoindolenine of the formula

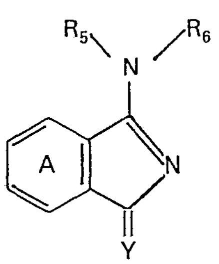

8

in which A and Y are as defined in claim 1 and $R_5$ and $R_6$ are H atoms or alkyl or heteroaryl radicals, or $R_5$ and $R_6$, together with the N atom, are a 5- to 6-membered heterocyclic ring, in the presence of metal donors, in a polar organic solvent.

5. Process according to claim 4, which comprises starting from an isoindolinone of the formula 7) or an isoindolenine of the formula 8) in which $R_5$ and $R_6$ are as defined in claim 4 and Y′ is a radical of the formula

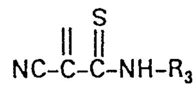

or

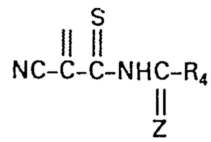

in which $R_3$, $R_4$ and Z are as defined in claim 2 or 3.

6. Process for colouring high-molecular organic material, which comprises using metal complexes according to claim 1.

7. High-molecular organic material containing a metal complex according to claim 1.

**Revendications**

1. Complexes métalliques 1:1 d'azines répondant à la formule (1):

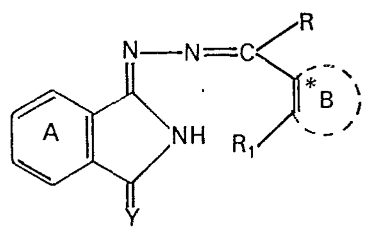

1

dans laquelle le noyau A peut porter d'autres substituants, R représente un atome d'hydrogène, un radical alkyle ou un radical aryle, B représente un radical aromatique isocyclique ou hétérocyclique ou un radical alicyclique, $R_1$ représente un radical -OH ou -SH et Y représente un radical répondant à la formule (2):

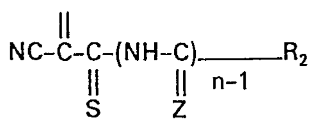

2

dans laquelle Z désigne un atome O ou S, n désigne le nombre 1 ou le nombre 2 et $R_2$ représente un radical alkyle, aralkyle, cycloalkyle ou aryle ou un radical amino éventuellement porteur d'un radical alkyle, cycloalkyle, aralkyle ou aryle.

2. Complexes métalliques selon la revendication 1 qui répondent à la formule (3):

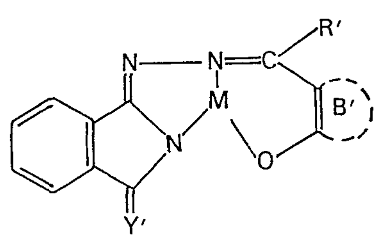

3

dans laquelle M représente le nickel ou le cuivre, R′ représente H ou un méthyle, B′ représente un radical de pyrazole, de pyridine, de pyrimidine, de quinoléine ou de coumarine et Y′ représente un radical répondant à la formule (4):

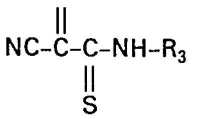

4

dans laquelle $R_3$ représente un atome d'hydrogène, un radical alkyle en $C_1$–$C_4$ ou un radical répondant à la formule (5):

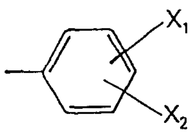

5

dans laquelle $X_1$ représente un atome d'hydrogène, de chlore ou de brome, un radical nitro, trifluorométhyle, carbamoyle ou sulfamoyle, un radical alkyle, alcoxy ou alkylsulfamoyle en $C_1$–$C_4$, un radical alcanoylamino, alkylcarbamoyle ou alcoxycarbonyle en $C_2$–$C_6$ ou un radical phénoxy, phénylcarbamoyle ou phénylsulfamoyle éventuellement porteur d'atomes de chlore ou de brome ou de radicaux méthylènes, et $X_2$ représente un atome d'hydrogène, de chlore ou de brome ou un radical alkyle ou alcoxy en $C_1$–$C_4$.

3. Complexes métalliques selon la revendication 1 dans lesquels Y représente un radical répondant à la formule

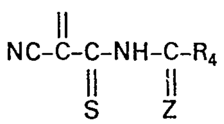

dans laquelle Z représente un atome O ou S et $R_4$ représente un radical alkyle en $C_1$–$C_4$, un radical cyclohexyle, un radical benzyle ou un radical répondant à la formule (5):

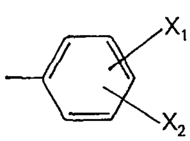

5

dans laquelle $X_1$ et $X_2$ ont les significations données à la revendication 2.

4. Procédé de préparation de complexes métalliques 1:1 selon la revendication 1, procédé caractérisé en ce que:

a) on traite une azine de formule (1) par des agents capables de céder un métal, ou

b) on chauffe en présence d'agents capables de céder un métal, dans un solvant organique polaire, une hydrazone répondant à la formule (6):

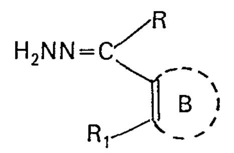

6

dans laquelle R, $R_1$ et B ont les significations données à la revendication 1, avec une iso-indolinone répondant à la formule (7):

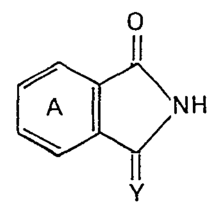

7

ou une amino-iso-indolénine répondant à la formule (8):

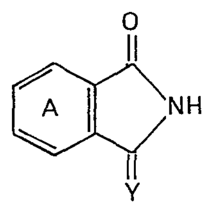

7

formules dans lesquelles A et Y ont les significations données à la revendication 1, $R_5$ et $R_6$ représentent chacun un atome d'hydrogène, un radical alkyle ou un radical hétéroaryle, ou encore $R_5$ et $R_6$ forment ensemble, et avec l'atome d'azote, un hétérocycle pentagonal ou hexagonal.

5. Procédé selon la revendication 4 caractérisé en ce qu'on part d'une iso-indolinone de formule (7) ou d'une isoindolénine de formule (8) dans lesquelles $R_5$ et $R_6$ ont les significations données à la revendication 4 et Y représente un radical répondant à l'une des formules:

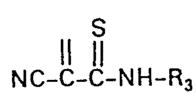

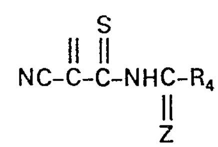

dans lesquelles $R_3$, $R_4$ et Z ont les significations données dans les revendications 2 et 3.

6. Procédé pour colorer des matières organiques à haut poids moléculaire, procédé caractérisé en ce qu'on utilise des complexes métalliques selon la revendication 1.

7. Matières organiques à haut poids moléculaire qui contiennent un complexe métallique selon la revendication 1.